Europäisches Patentamt
European Patent Office
Office européen des brevets

(11) Numéro de publication: **0 311 473 A1**

(19)

(12) # DEMANDE DE BREVET EUROPEEN

(21) Numéro de dépôt: **88402310.2**

(22) Date de dépôt: **14.09.88**

(51) Int. Cl.[4]: **C 07 C 101/10**
C 07 C 103/46, A 61 K 9/00,
A 61 K 47/00

(30) Priorité: **16.09.87 FR 8712815**

(43) Date de publication de la demande:
**12.04.89 Bulletin 89/15**

(84) Etats contractants désignés:
**AT BE CH DE ES FR GB GR IT LI LU NL SE**

(71) Demandeur: **APPLICATIONS ET TRANSFERTS DE TECHNOLOGIES AVANCEES ATTA**
**47, Corniche des Oliviers**
**F-06000 Nice (FR)**

(72) Inventeur: **Riess, Jean**
**Les Giaines**
**F-06950 Falicon (FR)**

**Blaignon, Christine**
**27, Rue Durand de Sartoux**
**F-06370 Mouans Sartoux (FR)**

**Le Blanc, Maurice**
**10, Avenue de Brancolar**
**F-06100 Nice (FR)**

(74) Mandataire: **Mongrédien, André et al**
**c/o BREVATOME 25, rue de Ponthieu**
**F-75008 Paris (FR)**

(54) **Nouveaux dérivés fluorés d'acides aminés, utilisables en particulier comme agents tensioactifs ou contensioactifs, et préparations à usage biomédical comprenant ces dérivés.**

(57) L'invention concerne de nouveaux dérivés fluorés d'acides aminés, utilisables en particulier comme agents tensioactifs ou cotensioactifs et préparation à usage médical comprenant des dérivés.

Ces dérivés fluorés sont de nouvelles molécules tensioactives et co-tensioactives biocompatibles, utilisables dans la formulation de préparations pharmaceutiques et vétérinaires, utilisables également en génie biologique et médical notamment dans des compositions jouant le rôle de transporteurs d'oxygène et d'autres gaz à base de composés hautement fluorés. Ces dérivés comportent, d'une part, une extrémité, ou "queue", hautement fluorée et, d'autre part, une autre extrémité, ou "tête", hydrophile dérivée d'un acide aminé, pris sous une forme neutre ou ionique, amphotère en particulier, ou lié à d'autres fragments tels que des stéroïdes, en particulier ceux appartenant à la famille des acides biliaires, ces deux extrémités étant reliées entre elles par des éléments de jonction dont la nature peut être modulée en fonction des propriétés recherchées pour chacun des composés considérés.

EP 0 311 473 A1

## Description

# NOUVEAUX DERIVES FLUORES D'ACIDES AMINES, UTILISABLES EN PARTICULIER COMME AGENTS TENSIOACTIFS OU COTENSIOACTIFS ET PREPARATIONS A USAGE BIOMEDICAL COMPRENANT CES DERIVES.

La présente invention a pour objet de nouveaux dérivés fluorés d'acides aminés, utilisables en particulier comme agents tensioactifs ou cotensioactifs.

De façon plus précise, elle concerne la réalisation d'agents tensioactifs ou cotensioactifs, biocompatibles, destinés à intervenir dans des préparations à usages pharmaceutique, vétérinaire ou phytosanitaire ou en génie biologique et médical, et en particulier dans des préparations destinées à jouer le rôle de transporteurs des gaz à usage biomédical.

On sait que la survie des tissus et organes du corps humain est rapidement compromise dès que l'approvisionnement en oxygène n'est plus assuré, comme en cas d'hémorragie ou d'anémie sévère ou encore d'ischémie cérébrale ou myocardiale.

Jusqu'à présent, le seul moyen efficace dont on disposait pour réaliser cet apport en oxygène, était la transfusion sanguine, mais toutes les situations pathologiques ou cliniques n'en relèvent pas. L'administration de sang ou de globules rouges est inappropriée en cas de thrombose cérébrale ou d'infarctus du myocarde par exemple, et la transfusion présente certains risques immunologiques et infectieux.

Aussi, des recherches ont été entreprises récemment pour mettre au point des transporteurs d'oxygène in vivo qui rempliraient non seulement les fonctions d'un concentré érythrocytaire mais seraient également utilisables dans des situations où l'administration des globules rouges est inefficace ou contre-indiquée.

Parmi les compositions destinés à jouer le rôle de transporteurs de gaz on peut citer également les solutions cardioplégiques et de reperfusion, les agents de diagnostic, les agents oxygénants non intravasculaires, par exemple pour la préservation de tissus isolés ou la perfusion par les voies ventriculosubarachnoïde péritonéale ou gastro-intestinale. Ces transporteurs d'oxygène peuvent simultanément remplir d'autres fonctions encore, comme celles d'agents de contraste en diagnostic ou de vecteurs de substances médicamenteuses.

Deux catégories de transporteurs d'oxygène peuvent être envisagées ; la première porte sur des composés dans lesquels l'oxygène est chimiquement coordiné comme c'est le cas avec l'hémoglobine ; la deuxième porte sur des composés dans lesquels l'oxygène est simplement dissous. Dans cette deuxième catégorie, on connaît des produits capables de réaliser le transport d'oxygène sous forme dissoute, ce sont les fluorocarbures qui sont à la fois les meilleurs solvants connus des gaz et parmi les composés les plus inertes que l'on sache préparer. Des produits de ce type sont en particulier décrits par Maurice LE BLANC et Jean RIESS dans "Preparation, Properties and Industrial Applications of Organofluorine Compounds", chap. 3, R.E. BANKS Ed., Ellis Horwood Ltd, Chichester, 1982. Cependant, leur introduction dans le système vasculaire ne peut être effectuée que sous la forme d'une émulsion car les fluorocarbures ne sont pas solubles dans l'eau. Une telle émulsion comprend l'agent transporteur d'oxygène à base de fluorocarbure, un ou plusieurs agents tensioactifs, de l'eau et d'autres ingrédients, par exemple des sels minéraux et un agent oncotique pour ajuster le pH et les pressions osmotique et oncotique, des agents cryoprotecteurs si l'émulsion doit être congelée. D'autres additifs peuvent encore y être ajoutés tels que des agents nutritifs, des vitamines, des stéroïdes, des prostaglandines, des antibiotiques, des agents thrombolytiques, en fonction des indications thérapeutiques.

Dans de telles émulsions, les fluorocarbures sont dispersés sous la forme de gouttelettes ayant environ 0,1 µm grâce à un ou plusieurs agents tensioactifs.

A titre d'exemple, on connaît une émulsion de fluorocarbure à usage biomédical : le Fluosol-DA 20%, développée par la Green Cross Corp au Japon, qui est décrite dans la publication citée plus haut.

Les émulsions de ce type présentent certains inconvénients et certaines limitations qui sont décrits dans un article de Jean RIESS paru dans la Revue Française de Transfusion et Immuno-hématologie, tome XXIX, p.423-441, 1986, et dans un Rapport Technique présenté à un Symposium de l'ONUDI sur le Sang et ses Dérivés et publié par la Fondation Marcel Mérieux en mai 1987. En effet, les tensioactifs utilisés sont polydisperses et mal définis. De plus l'un d'eux, le Pluronic F-68 provoque une réaction anaphylactique transitoire chez certains patients ; la stabilité de ces émulsions est limitée et il faut les congeler pour les conserver ; elles ne sont pas prêtes à l'emploi puisqu'il faut mélanger trois préparations, l'émulsion mère et deux solutions annexes, avant de les administrer ; enfin, leur efficacité est doublement limitée, d'une part, à cause de leur faible concentration en fluorocarbures (20% en poids ne représente que 11% en volume), conduisant pour les doses habituellement utilisées à un taux de fluorocarbures dans le sang d'environ 3% seulement, et d'autre part, du fait de leur persistance intravasculaire limitée ; enfin il est difficile d'adapter leurs caractéristiques en fonction de l'application spécifique recherchée.

On peut dire d'une manière plus générale que les propriétés des agents tensioactifs connus et utilisés jusqu' à ce jour sont encore insuffisantes pour permettre de bien maîtriser les caractéristiques de ces émulsions, et notamment leur persistance intravasculaire et leur stabilité, et d'en adapter les caractéristiques à une application thérapeutique donnée.

Aussi, des recherches ont été effectuées pour améliorer les caractéristiques et performances de ces émulsions, et elles ont porté en particulier sur la mise au point de nouveaux agents tensioactifs et/ou

cotensioactifs, biocompatibles qui soient mieux adaptés à l'émulsification des fluorocarbures que les agents tensioactifs actuellement utilisés.

La présente invention a précisément pour objet de nouveaux dérivés fluorés d'acides aminés, qui sont des agents tensioactifs susceptibles d'être utilisés dans de telles émulsions pour pallier les inconvénients des agents tensioactifs actuellement utilisés.

Ces nouveaux dérivés sont des molécules qui présentent la particularité de comporter, d'une part, une extrémité hautement fluorée et "fluorophile", c'est-à-dire qui présente une forte affinité pour les phases hautement fluorées ou perfluorées et, d'autre part, une extrémité hydrophile dérivée d'un acide aminé naturel, neutre ou ionique, et amphotère en particulier, extrémité hydrophile qui peut encore être transformée en un dérivé de bétaïne ou modifiée par association avec un autre fragment comme ceux dérivés des acides biliaires et des stéroïdes, ces deux extrémités qui sont respectivement fluorophile et hydrophile, étant réunies par une chaîne dont la structure peut être modulée en fonction des propriétés recherchées.

Selon l'invention, ces nouveaux dérivés fluorés d'acides aminés répondent aux formules :

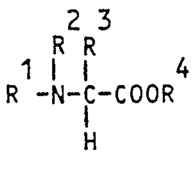
ou
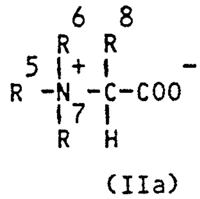

(I) (IIa)

dans lesquelles :

- $R^1$ est un atome d'hydrogène, un radical alkyle de 1 à 24 atomes de carbone, un radical dérivé d'un stéroïde ou un radical fluoré de formule $R_F$-$(CH_2)_p$- ou de formule $R_F$-$(CH_2)_p$-C(O)- dans lesquelles p est un nombre entier de 1 à 24, et $R_F$ représente un radical aliphatique perfluoré linéaire ou ramifié, comportant éventuellement dans sa chaîne 1 ou plusieurs atomes d'oxygène, certains atomes de fluor du radical perfluoré pouvant être remplacés par un ou plusieurs substituants choisis parmi les atomes d'hydrogène, de chlore et de brome à condition que le nombre d'atomes de fluor du radical $R_F$ soit supérieur à 7 et que le groupe terminal du radical $R_F$ situé à l'extrémité opposée de l'extrémité par laquelle le radical $R_F$ est relié à $CH_2$ ne comporte pas d'atome d'hydrogène ;
- $R^2$ représente un atome d'hydrogène ou un radical alkyle de 1 à 24 atomes de carbone ;
- $R^3$ représente un atome d'hydrogène, un radical de formule : $H_2N$-C(=NH)-NH-$(CH_2)_3$- ou un radical fluoré de formule :

$R_F$-$(CH_2)_p$-, $R_F$-$(CH_2)_n$-C(O)-, $R_F$-$(CH_2)_n$-$X^1$-$(CH_2)_m$-,
$R_F$-$(CH_2)_p$-O-C(O)-$(CH_2)_m$- ou $R_F$-$(CH_2)_n$-$X^2$-CH($R^9$)-

dans lesquelles :

- $R_F$ et p ont la signification donnée ci-dessus, m est un nombre entier de 1 à 4, n est égal à 0 ou est un nombre entier de 1 à 24, $X^1$ représente 0 ; S ; -C(O)-NH- ; -C(O)-O- ; $X^2$ représente -0- ou -C(O)-O-, et $R^9$ représente un radical alkyle de 1 à 6 atomes de carbone ou un radical aryle de 6 à 10 atomes de carbone ;
- $R^4$ représente un atome d'hydrogène, un radical alkyle ou un métal physiologiquement acceptable ;
- $R^5$, $R^6$, $R^7$ et $R^8$ qui peuvent être identiques ou différents, représentent un atome d'hydrogène, un radical alkyle ayant de 1 à 24 atomes de carbone ou un radical fluoré de formule :

$R_F$-$(CH_2)_p$-, $R_F$-$(CH_2)_n$-C(O)-, $R_F$-$(CH_2)_n$-$X^1$-$(CH_2)_m$-
$R_F$-$(CH_2)_p$-O-C(O)-$(CH_2)_m$- ou $R_F$-$(CH_2)_n$-$X^2$-CH($R^9$)-

dans lesquelles $R_F$, p, n, m, $X^1$, $X^2$ et $R^9$ ont la signification donnée ci-dessus,
à condition que l'un au moins des radicaux $R^1$ et $R^3$ dans la formule (I) et l'un au moins des radicaux $R^5$, $R^6$ et $R^7$ et $R^8$ dans la formule (IIa) soit un radical fluoré.

Les nouveaux dérivés de l'invention ont en particulier l'avantage d'être des agents tensioactifs ou des précurseurs d'agents tensioactifs monodisperses qui peuvent être préparés sous une forme hautement purifiée. Par ailleurs, la présence d'un groupe fluorophile est mieux adaptée pour réaliser l'émulsification des fluorocarbures tels que ceux utilisés comme agents transporteurs d'oxygène. De plus, ces dérivés sont faciles à réaliser industriellement et sont biocompatibles en particulier hémocompatibles. Aussi, ils présentent un grand intérêt pour la réalisation d'émulsions contenant des agents transporteurs d'oxygène à base de fluorocarbures, utilisables en particulier comme substituts du sang et du plasma, milieux du traitement de l'ischémie, solutions cardioplégiques et de reperfusion, agents de diagnostic, agents oxygénants non intravasculaires, par exemple pour la préservation de tissus isolés ou la perfusion par voie ventriculo-subarachnoïde, péritonéale ou gastrointestinale.

Dans l'invention, on entend par groupe perfluoré un groupe aliphatique dans lequel tous les atomes d'hydrogène ont été remplacés par du fluor, par exemple un radical alkyle perfluoré de formule $C_nF_{2n+1}$-.

Dans les dérivés de l'invention, ce radical perfluoré peut comporter dans sa chaîne un ou plusieurs atomes d'oxygène. Toutefois, dans les radicaux fluorés utilisés dans l'invention, certains atomes de fluor du radical perfluoré peuvent être remplacés par des substituants choisis parmi les atomes d'hydrogène, de chlore et de

brome. Dans ce dernier cas, on effectue plutôt cette substitution en position terminale de la chaîne. Par ailleurs, pour obtenir les propriétés recherchées, en particulier le caractère fluorophile, il est nécessaire que le radical fluoré comprenne un nombre d'atomes de fluor supérieur à 7.

Les radicaux fluorés susceptibles d'être utillisés dans l'invention sont donc du type :

$R_F-(CH_2)_p-C(O)-$
$R_F-(CH_2)_p-$
$R_F-(CH_2)_n-C(O)-$
$R_F-(CH_2)_n-O-(CH_2)_m-$
$R_F-(CH_2)_n-S-(CH_2)_m-$
$R_F-(CH_2)_p-O-C(O)-(CH_2)_m-$
$R_F-(CH_2)_n-C(O)-NH-(CH_2)-_m$
$R_F-(CH_2)_n-C(O)-O-(CH_2)-_m$
$R_F-(CH_2)_n-O-CHR^9-$
$R_F-(CH_2)_n-C(O)-O-CHR^9-$

A titre d'exemple de radicaux $R_F$ susceptibles d'être utilisés dans les radicaux fluorés mentionnés ci-dessus, on peut citer les radicaux de formules suivantes :

$F(CF_2)_v-$ avec $4 \leqq v \leqq 12$ ;
$(CF_3)_2CF(CF_2)_w-$ avec $1 \leqq w \leqq 8$ ;
$CF_3-[CF_2CF(CF_3)]_r-$ avec $1 \leqq r \leqq 4$ ;
$C_2F_5-[CF_2CF(CF_3)]_r-$ avec $1 \leqq r \leqq 4$ ;
$(CF_3)_2[CF-CF_2CF(CF_3)]_r-$ avec $1 \leqq r \leqq 4$ ;

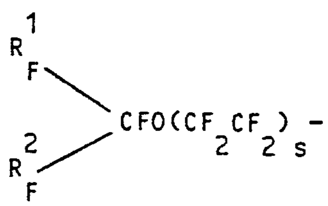

dans laquelle $1 \leqq s \leqq 6$ et $R_F^1$ et $R_F^2$, qui sont identiques ou différents, sont choisis parmi $CF_3-$, $C_2F_5-$, $n-C_3F_7-$, et $CF_3CF_2CF(CF_3)-$
ou dans laquelle $R_F^1$ et $R_F^2$ forment ensemble un radical bivalent choisi parmi $-CF_2(CF_2)_2CF_2-$ et $-CF_2(CF_2)_3CF_2-$ ;
$CF_3CF_2O-(CF_2CF_2O)_t-CF_2-$ avec $0 \leqq t \leqq 6$ ; et
$CF_3(CF_2)_2O-[CF(CF_3)(CF_2O)]_u-CF(CF_3)-$ avec $0 \leqq u \leqq 6$.

Les dérivés fluorés de l'invention ont des structures différentes selon que le radical fluoré est accroché à l'atome d'azote ou à l'atome de carbone du motif aminoacide.

Selon un premier mode de réalisation de l'invention, le radical fluoré est accroché à l'atome de carbone du motif aminoacide.

A titre d'exemple de tels dérivés, on peut citer ceux répondant aux formules : (dans toutes ces formules $R^{10}$ est le radical $F(CF_2)_v-$ avec $4 \leqq v \leqq 12$, $0 \leqq n \leqq 24$ et $1 \leqq p \leqq 24$.

1°) 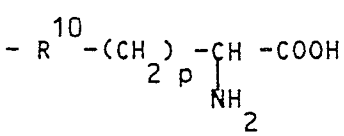 (III)

2°) 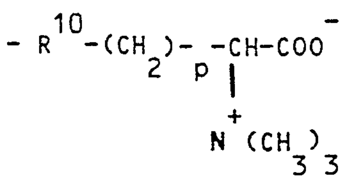 (IV)

3°) $-R^{10}-(CH_2)_n-C(O)-N(R^{11})-CH_2-COOR^4$ (V)
dans laquelle $R^{11}$ représente un atome d'hydrogène ou un radical alkyle, de préférence $CH_3$, et $R^4$ représente un atome d'hydrogène ou un métal physiologiquement acceptable.

4°) $-R^{10}-(CH_2)_p-O-C(O)-(CH_2)_m-\underset{NH_2}{\underset{|}{C}}H-COOH$ (VI)

dans laquelle m est un nombre entier de 1 à 4.

5°) $-R^{10}-(CH_2)_n-C(O)-NH-(CH_2)_m-\underset{NH_2}{\underset{|}{C}}H-COOH$ (VII)

dans laquelle m est un nombre entier de 1 à 4.

6°) $-R^{10}-(CH_2)_n-C(O)-NH-(CH_2)_m-\underset{\overset{+}{N}(CH_3)_3}{\underset{|}{C}}H-COO^-$ (VIII)

dans laquelle m est un nombre entier de 1 à 4.

7°) $-R^{10}-(CH_2)_p-\underset{NHR_{12}}{\underset{|}{C}}HCOOR^4_{12}$ (IX)

dans laquelle R représente le radical cholyle ou un radical dérivé ou apparenté à celui-ci, et $R^4$ un cation approprié tel que par exemple $Na^+$.

8°) $R^{10}(CH_2)_pN^+(CH_3)_2-CH_2-COO^-$ (X)

Les dérivés qui comportent le radical fluoré sur l'atome de carbone du motif aminoacide, peuvent être préparés par des procédés classiques qui dépendent en particulier de la nature de substituant fluoré utilisé.

A titre d'exemple, on donne ci-après, le mode de préparation des dérivés fluorés de formule III, IV, VI, VII et VIII.

1°) Dérivés fluorés de formule III :

On peut préparer ces dérivés en partant du diphénylméthylidène aminoacétate d'éthyle que l'on fait réagir avec l'iodure du radical fluoré correspondant $R^{10}-(CH_2)_pI$ pour former le diphénylméthylidène aminobutanoate d'éthyle substitué par ce radical fluoré selon :

$$R^{10}-(CH_2)_pI + (Ph)_2C=N-CH_2-COOH \longrightarrow R^{10}-(CH_2)_p-\underset{N=C(Ph)_2}{\underset{|}{C}}H-COOEt$$

Après cette réaction, on forme le chlorhydrate du dérivé fluoré correspondant en traitant par de l'acide chlorhydrique le produit obtenu précédemment, puis on fait réagir ce chlorhydrate avec de la soude pour former le dérivé fluoré de formule III désiré. Ceci correspond aux schémas réactionnels suivants :

$$R^{10}-(CH_2)_p-\underset{\substack{|\\ N=C(Ph)_2}}{CH}-COOEt \xrightarrow{HCl} R^{10}-(CH_2)_p-\underset{\substack{|\\ NH_2.HCl}}{CH}-COOEt \xrightarrow{NaOH} (III)$$

Les produits de départ utilisés pour cette synthèse sont des produits du commerce ou peuvent être préparés par des techniques classiques. Ainsi, certains iodures $R^{10}(CH_2)_pI$ sont commerciaux ou peuvent être préparés par des méthodes bien connues, le diphénylméthylidène aminoacétate d'éthyle est préparé à partir du glycinate d'éthyle et de la benzophénone selon une méthode proposée par O'Donnell et Polt dans J. Org. Chem. 47, 2663, 1982.

2°) Dérivés fluorés de formule IV.

On peut former ces dérivés de formule IV en faisant réagir l'acide de formule III avec divers agents alkylants dont l'iodure de méthyle en présence de bicarbonate de sodium selon :

$$R^{10}(CH_2)_p-\underset{\substack{|\\ NH_2}}{CH}-COOH + CH_3I \longrightarrow R^{10}(CH_2)_p-\underset{\substack{|+\\ N(CH_3)_3}}{CH}-COO^- \quad (IV)$$

3°) Dérivés fluorés de formule VI :

On peut préparer ces dérivés fluorés en faisant réagir le diacide de formule :

$$HOOC(CH_2)_m-\underset{\substack{|\\ NH_2}}{CH}-COOH$$

avec l'alcool fluoré correspondant de formule $R^{10}(CH_2)_pOH$. Ceci correspond à la réaction ci-dessous :

$$R^{10}(CH_2)_pOH + HOOC(CH_2)_m-\underset{\substack{|\\ NH_2}}{CH}-COOH \longrightarrow (VI)$$

Cette réaction peut être effectuée dans l'acide chlorhydrique concentré au reflux.

Les diacides de départ de cette réaction sont des acides aminés naturels et certains des alcools fluorés de formule $R^{10}(CH_2)_pOH$ sont commerciaux, les autres peuvent être préparés par des méthodes bien connues de l'homme de l'art.

4°) Dérivés fluorés de formule (VII).

On peut préparer ces dérivés fluorés en faisant réagir l'acide fluoré de formule $R^{10}(CH_2)_nCOOH$ avec un aminoacide de formule :

$$NH_2-(CH_2)_m-\underset{\substack{|\\ NH_2}}{CH}-COOH.$$

dans laquelle m est un nombre entier de 1 à 4, de préférence 4.

Ceci correspond au schéma réactionnel suivant :

$$R^{10}-(CH_2)_n-COOH + H_2N-(CH_2)_m-\underset{\displaystyle NH_2}{\underset{|}{CH}}-COOH \rightarrow (VII)$$

Cette réaction peut être effectuée à la température ambiante en utilisant un solvant organique tel que l'éther. Le sel formé est alors séparé, chauffé à 150°C pour conduire au composé VII.

Les produits de départ utilisés pour cette réaction sont d'une part des acides aminés naturels tels que la lysine, et d'autre part des acides fluorés commerciaux ou qui peuvent être préparés par des méthodes connues.

5°) Dérivés fluorés de formule VIII.

Les dérivés fluorés de formule VII peuvent être convertis en dérivés de bétaïne (VIII) par réaction avec divers agents alkylants comme l'iodure de méthyle et en présence de bicarbonate de potassium.

Lorsque le radical fluoré est accroché à l'atome d'azote du motif aminoacide on peut également obtenir les dérivés fluorés correspondants par des méthodes classiques. A titre d'exemple, on donne ci-après le mode de préparation des dérivés de formule V.

6°) Dérivés fluorés de formule (V).

On peut obtenir ces dérivés fluorés par réaction du chlorure d'acide $R^{10}$-$(CH_2)_n$-COCl sur le chlorhydrate de l'ester éthylique de formule :

$R^{11}$-NH-$CH_2$-COOEt, HCl

pour former l'ester de formule :

$R^{10}$-$(CH_2)_n$-C(O)-N($R^{11}$)-$CH_2$-COOEt

où $R^{10}$, n et $R^{11}$ ont la signification donnée ci-dessus, que l'on transforme ensuite en l'acide correspondant de formule (V) par saponification à la soude puis neutralisation à l'acide chlorhydrique.

Les esters de départ peuvent être préparés à partir des acides aminés correspondants tels que la glycine ou la sarcosine. Les chlorures d'acide peuvent être préparés par action du chlorure de thionyle sur les acides correspondants.

7°) Dérivés fluorés de formule (X)

On peut préparer ces dérivés fluorés en partant de l'iodure du radical fluoré correspondant $R^{10}(CH_2)_pI$ que l'on transforme en azide $R^{10}(CH_2)_pN_3$ puis en l'amine $R^{10}(CH_2)_pNH_2$ et en l'amine $R^{10}(CH_2)_pN(CH_3)_2$. On fait ensuite réagir cette amine avec du chloroacétate d'éthyle pour former le chlorure $R^{10}(CH_2)_pN^+(CH_3)_2CH_2COOC_2H_5,Cl^-$ que l'on convertit en dérivé de formule (X).

Les dérivés fluorés de l'invention peuvent être utilisés comme agents tensioactifs ou cotensioactifs dans des préparations à usages pharmaceutique, vétérinaire ou phytosanitaire, et dans des préparations utilisables en génie biologique et médical comprenant en particulier des transporteurs d'oxygène du type fluorocarbures et/ou d'autres composés hautement fluorés.

Aussi, la présente invention a également pour objet des préparations, solutions, dispersions, gels, émulsions et microémulsions dans l'eau ou tout autre solvant polaire, de substances et composés non polaires en particulier hautement fluorés ou perfluorés, souvent appelés fluorocarbures, qui comprennent au moins l'un des dérivés fluorés de l'invention.

A titre d'exemple, les composés hautement fluorés ou perfluorés concernés sont des composés linéaires ou cycliques, ayant des masses moléculaires allant de préférence de 400 à 700 et ils peuvent être choisis par exemple parmi les composés suivants :

les bis(F-alkyl)-1,2-éthènes et plus particulièrement les bis(F-butyl)-1,2-éthènes, les F-isopropyl-1-F-hexyl-2-éthènes et les bis(F-hexyl)-1,2-éthènes, les perfluorodécalines, les perfluorométhyldécalines, les perfluoro-diméthyldécalines, les perfluorométhyl- et diméthyladamantanes, les perfluorodi- et triméthylbicyclo(3,3,1)nonanes et leurs homologues, les éthers de formule :

$(CF_3)_2CFO(CF_2CF_2)_2OCF(CF_3)_2$, $(CF_3)_2CFO(CF_2CF_2)_3OCF(CF_3)_2$,

$(CF_3)_2CFO(CF_2CF_2)_2F$, $(CF_3)_2CFO(CF_2CF_2)_3F$,

F $[CF(CF_3)CF_2O]_2CHFCF_3$, F $[CF(CF_3)CF_2O]_3CHFCF_3$, $(C_6F_{13})_2O$,

les amines $N(C_3F_7)_3$, $N(C_4F_9)_3$, les perfluorométhylquinolidines et perfluorométhylisoquinolidines, les dérivés halogénés $C_6F_{13}Br$, $C_8F_{17}Br$, $C_6F_{13}CBr_2CH_2Br$, bromo-1 perfluoroisopropyl-4 cyclohexane.

Ces composés hautement fluorés peuvent être utilisés seuls ou en mélange.

Dans ces préparations, solutions, émulsions, gels, dispersions ou microémulsions comprenant un ou plusieurs des composés hautement fluorés mentionnés ci-dessus, on peut utiliser des dérivés fluorés de

l'invention en les associant à un ou plusieurs autres agents tensioactifs fluorés ou non fluorés tels que, par exemple, les phospholipides ou les agents du type polyoxyéthylène/polyoxypropylène tels que ceux commercialisés sous les marques Pluronic ou Symperonic.

Les préparations, solutions, gels, émulsions, dispersions ou microémulsions de l'invention sont plus particulièrement destinées à servir de transporteurs de gaz, et en particulier de l'oxygène, en milieu vivant, pour des applications en médecine humaine et vétérinaire et en biologie, en particulier comme substituts du sang, agents de contraste pour le diagnostic, milieux pour le traitement de l'ischémie cérébrale et cardiaque, pour la préservation d'organes, de tissus, d'embryons, de semences, milieux utilisables en thérapeutique et chirurgie cardiovasculaire, par exemple comme solution cardioplégique, ou de reperfusion, ou en angioplastie coronaire, milieux utilisables comme adjuvant en radiothérapie et chimiothérapie du cancer, milieux utilisables comme vecteurs de médicaments.

D'autres caractéristiques et avantages de l'invention apparaîtront mieux à la lecture des exemples suivants, donnés bien entendu à titre illustratif et non limitatif.

EXEMPLE 1 : Acide amino-2 F-butyl-4 butanoïque

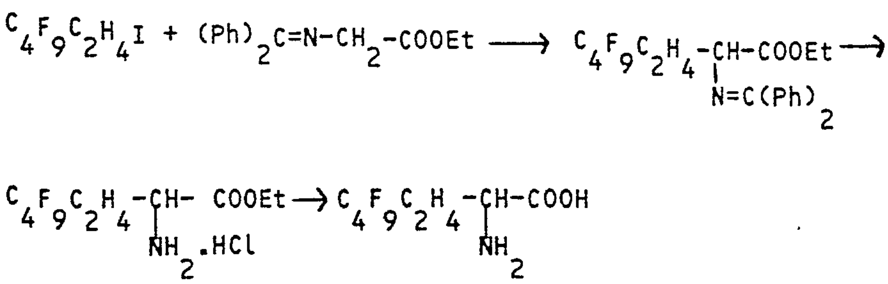

Etape 1 : Préparation du diphénylméthylidène amino-2 F-butyl-4 butanoate d'éthyle (composé n°1)

7 ml (11 mM) de butyllithium 1,6 M dans l'hexane sont ajoutés sous argon ou azote à 2,1 ml (15 mM) de diisopropylamine dans 20 ml de tétrahydrofuranne (THF) anhydre à 25°C. Après 30 minutes, le mélange réactionnel est refroidi à -70°C et 2,7 g (10,6 mM) de diphénylméthylidèneaminoacétate d'éthyle sont additionnés. Il se forme un précipité qui est dissous par addition de 1 ml d'oxyde de trisdiméthylaminophosphine anhydre. Le mélange obtenu est ajouté à 25°C, sous argon ou azote à 7,92 g (21,2 mM) d'iodo-1(F-butyl)-2 éthane dans le THF anhydre. Après 15 heures, le THF est évaporé sous pression réduite et le résidu est dissous dans l'éther. La phase organique est lavée trois fois à l'eau et séchée sur sulfate de magnésium anhydre, filtrée, et distillée. Le composé 1 en mélange avec de la benzophénone est séparé par chromatographie sur silice. Ce mélange est utilisé tel quel dans la deuxième étape.

L'analyse par spectrométrie infrarouge (IR) et par résonance magnétique nucléaire (RMN) du mélange donne les résultats suivants :

IR ($\nu cm^{-1}$, film) : 1670 ; 1450 ; 1280 ; 1320,

RMN$^1$H (δppm, $CDCl_3$, TMS) : 1,12 (t, 3H) ; 2,2 (m, 4H) ; 4,2 (q+t, 3H) ; 7,5 (m, H aromatiques).

Etape 2 : Préparation du chlorhydrate d'amino-2 F-butyl-4 butanoate d'éthyle (composé n°2)

Le mélange issu de l'étape 1, en solution dans l'éther éthylique est traité par 1,5 équivalent de HCl 1N à 25°C pendant 5 heures. La phase aqueuse est ensuite lavée trois fois à l'éther. Après évaporation, on obtient 1,35 g du composé n°2 (Rdt 33% par rapport au diphénylméthylidèneaminoacétate d'éthyle).

F : 104°C

IR ($\nu cm^{-1}$, KBr) : 1750, 1200

RMN$^1$H(δ ppm, $CD_3OD$, TMS) : 1,35 (t, 3H) ; 2,3 (m, 4H); 4,35 (q+t, 3H)

RMN$^{19}$F (δ ppm, $CD_3OD$, $CCl_3f$) : 114 ($-CF_2CH_2$)

SM (LID/IC $CH_4$) m/e (%) : M-Cl ; 350 (100) ; 350-HF : 330(31) $H_2N-CH-C_2H_4C_4F_9$ : 276(92).

Etape 3 : Préparation de l'acide amino-2 F-butyl-4 butanoïque (composé n° 3)

Le chlorhydrate de l'amino-2 F-butyl-4 butanoate d'éthyle est dissous dans un minimum d'eau et 2,3 équivalents de NaOH 10% sont ajoutés. Le mélange réactionnel est chauffé à reflux durant une nuit. Après neutralisation par HCl 1N, l'acide amino-2 F-butyl-4 butanoïque est isolé par filtration (Rdt 95%) et purifié par sublimation (175°C/18 mmHg).

F : sublime à 230°C,

Microanalyse : C trouvé (calculé) : C 29,9% (29,92) ; H 2,46 (2,51) ; F 53,41 (53,24); N 4,28 (4,36)

IR ($\nu$ $cm^{-1}$, KBr) : 1600, 1520, 1230, 1148

SM (LID/IC $NH_3$) m/e(%) : M+1 322 (16,6) : M 321 (10,9)

EXEMPLE 2 : Acide amino-2 F-hexyl-4 butanoïque (composé n°6)

$$C_6F_{13}C_2H_4-\underset{NH_2}{CH}-COOH$$

On répète le processus décrit à l'exemple 1, en faisant réagir dans la première étape 10g (39,2 mM) de diphénylméthylidène amino acétate d'éthyle avec 47,1 g (99 mM) d'iodo-1 (F-hexyl)-2 éthane. Le mélange de diphénylméthylidène amino-2 F-hexyl-4 butanoate d'éthyle (composé n°4) et de benzophénone ainsi obtenu donne dans la seconde étape 47 g de chlorhydrate d'amino-2 F-hexyl-4 butanoate d'éthyle (composé n°5) (Rdt 25%).
F : 115°C
Microanalyse : C trouvé (calculé) : C 30,15% (29,67) ; H 2,77 (2,70) ; F 49,76 (50,85); N 2,85 (2,88) ; Cl 7,4 (7,3)
IR ($\nu$ $cm^{-1}$, KBr) : 1750 ; 1200
RMN$^{19}$F ($\delta$ ppm, $CD_3OD$, $CCl_3F$) : -114 ($CF_2CH_2$)
RMN$^{1}$H($\delta$ppm, $CD_3OD$, TMS) : 1,35(t,3H); 2,27(m,4H); 4,35(t+q,3H)
SM (LID/IC $NH_3$) m/e(%) : M-Cl 450 (100)

Enfin, dans une troisième étape, en partant de 4 g (8,23 mM) de chlorhydrate d'amino-2 F-hexyl-4 butanoate d'éthyle (composé n°5), on obtient 3,3 g (95%) d'acide amino-2 F-hexyl-4 butanoïque (composé n°6).
F : sublime à 230°C
Microanalyse : C trouvé (calculé) : C 28,92 (28,52) ; H 1,96 (1,91) ; F 58,18 (58,64) ; N 3,27 (3,32)
IR ($\nu cm^{-1}$, KBr) : 1600 - 1520
SM (LID/IE) m/e (%) : M-COOH 376 (100)

EXEMPLE 3 : Acide amino-2 F-octyl-4 butanoïque (composé n°9)

$$C_8F_{17}C_2H_4-\underset{NH_2}{CH}-COOH$$

Le processus décrit dans l'exemple 1 est répété avec 5,1 g (20 mM) de diphénylméthylidène amino acétate d'éthyle et 34,5 g (60 mM) d'iodo-1 (F-octyl)-2 éthane. Le mélange de diphénylméthylidène amino-2 F-butyl-4 butanoate d'éthyle (composé n°7) et de benzophénone donne dans la seconde étape 2,10 g de chlorhydrate d'amino-2 F-octyl-4 butanoate d'éthyle (Rdt 18%) (comosé n°8).
F : 120°C
IR ($\nu$ $cm^{-1}$, KBr) : 1750, 1200
RMN$^{1}$H($\delta$ppm, $CD_3OD$, TMS) : 1,30(t,3H); 2,30(m,4H); 4,35(q+t,3H)
RMN$^{19}$F$\delta$($\delta$ ppm, $CD_3OD$, $CCl_3F$) : -114 ($CF_2CH_2$)
SM (LID/IE) m/e(%) : M-Cl 550 (10) ; $C_8F_{17}C_2H_4CH=NH_2$ 476 (100)

Dans la troisième étape en utilisant 2g (3,41 mM) de chlorhydrate d'amino-2 F-octyl-4 butanoate d'éthyle (composé n°8), on obtient 1,68 g (95%) d'acide amino-2 F-octyl-4 butanoïque (composé n°9).
F : sublime à 230°C
Microanalyse : C trouvé (calculé) : C 27,71 (27,65) ; H 1,47 (1,55) ; F 61,28 (61,97) ; N 2,87 (2,68)
IR ($\nu$ $cm^{-1}$, KBr) : 1600, 1500, 1200
SM (LID/IC $NH_3$) m/e(%) : M+1 522 (3,2) ; M-COOH 476 (3,9)

EXEMPLE 4 : F-(butyl-2')éthyl bétaïne (composé n°10)

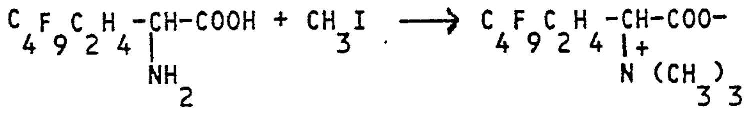

(Le motif triméthylammonioacétate $(CH_3)_3^+N\text{-}CH\text{-}COO^-$ sera considéré comme chaîne principale et systématiquement désigné par le nom bétaïne).

1 g (3,12 mM) d'acide amino-2 F-butyl-4 butanoïque (composé n° 3 obtenu dans l'exemple 1) dans 50 ml de méthanol est traité par 3,12 g (31,1 mM) de bicarbonate de sodium et par 3,12 ml (50,1 mM) d'iodure de méthyle. Le mélange réactionnel est agité à la température ambiante pendant 24 heures. Après évaporation à sec, le résidu est lavé au chloroforme, puis neutralisé avec HCl 0,1N. L'eau est évaporée lentement et la (F-butyl-2')éthylbétaïne (composé n° 10) est extraite à l'acétone. Après purification par HPLC (chromatographie liquide à haute performance) préparative 0,88 g de (F-butyl-2')éthylbétaïne (composé n° 10) sont obtenus (Rdt 78%).
F : 218° C
Microanalyse : C trouvé (calculé) : C36,17 (36,37) ; H 4,38 (3,88) ; F 44,84 (47,07) ; N 3,27 (3,85)
IR (ν $cm^{-1}$, KBr) : 1634, 1366
RMN$^1$H (δ ppm, $D_2O$, TMS) : 3,28 (S, 9H); 2,27 (m, 4H) ; 3,77 (t, 1H)
RMN$^{19}$F (δ ppm, $CD_3OD$, $CCl_3F$) : -114 ($\underline{C}F_2CH_2$)
SM (LID/IE) m/e(%) : M+1 : 364 (41,3) , $\overline{M}$-$NMe_3$ : 304 (94,4) ; M-$CH_2CH_2C_4F_9$ : 116 (26)

EXEMPLE 5 : (F-hexyl-2')éthyl bétaïne (composé n° 11)

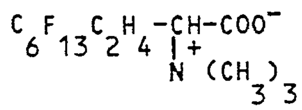

Le processus décrit dans l'exemple 4 appliqué à 1 g (2,37 mM) d'acide amino-2 F-hexyl-4 butanoïque (composé n° 6) obtenu dans l'exemple 2, conduit à 700 mg (64%) de (F-hexyl-2')éthyl bétaïne (composé n° 11).
F : 210° C
Microanalyse : C trouvé (calculé) : C 33,12 (33,70) ; H 3,04 (3,05) ; F 52,62 (53,32) ; N 2,88 (3,02)
IR (ν $cm^{-1}$, KBr) : 1632, 1362, 1200
RMN$^1$H (δ ppm, $CD_3OD$, TMS) : 3,30 (s, 9H) ; 2,27 (m, 4H) ; 3,77 (t, 1H)
RMN$^{19}$F (δ ppm, $CD_3OD$, $CCl_3F$) : -114 (-$\underline{C}F_2CH_2$)
SM (LID/IE) m/e (%) : M+1 464 (59,5) ; $\overline{M}$-$NMe_3$ 403 (84,5)

EXEMPLE 6: (F-octyl-2')éthyl bétaïne (composé n° 12)

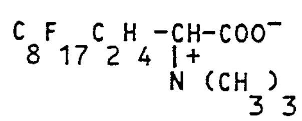

On répète le processus décrit dans l'exemple 4 sur 1g (1,77 mM) d'acide amino-2 F-octyl-4 butanoïque (composé n° 9) obtenu dans l'exemple 3 et on obtient 595 mg (55%) de (F-octyl-2')éthyl bétaïne (composé n° 12).
F : 215° C
Microanalyse : C trouvé (calculé) : 30,72 (31,98) ; H 2,54 (2,50) ; F 54,24 (57,34) ; N 2,24 (2,48)
IR (ν $cm^{-1}$, KBr) : 1630, 1370
RMN$^1$H (δ ppm, $CD_3OD$, TMS) : 3,3 (s, 9H) ; 2,27 (m, 4H) ; 3,77 (t, 1H)

RMN[19]F ($\delta$ ppm, $CD_3OD$, $CCl_3F$) : -114 (-$\underline{CF_2}CH_2$)
SM (LID/IC $NH_3$) m/e (%) : M+1 564 (100) ; M-$NMe_3$ 504 (59,7)

EXEMPLE 7 : (N,N,N-triméthylamonio)-2 (F-octyl)-3 propanoate (composé n°18).
$C_8F_{17}C_2H_4COOH$ ⟶ $C_8F_{17}CH_2CHBrCOOH$ (composé n°13), ⟶ $C_8F_{17}CH_2CHBrCOOEt$ (composé n°14), ⟶ $C_8F_{17}CH_2CH(N_3)COOEt$ (composé n°15), ⟶ $C_8F_{17}CH_2CH(NH_2)COOEt$, HCl (composé n°16), ⟶ $C_8F_{17}CH_2CH$ $[N^+(CH_3)_3]$ COOEt, Cl (composé n°17), ⟶ $C_8F_{17}CH_2CH$ $[N^+(CH_3)_3]$ $COO^-$ (composé n°18).

Etape 1 : Préparation d'acide bromo-2 (F-octyl)-3 propanoique (composé n°13).

On scelle sous vide dans une ampoule 41 g (83,33 mM) d'acide (F-octyl)-3 propanoïque, 5,2 ml (100mM) de brome et 0,2 ml de $PCl_3$ et on chauffe à 150°C pendant 48 heures. Après évaporation de HBr, le solide obtenu est dissous dans de l'éther éthylique. La phase organique est lavée avec 30 ml de solution aqueuse saturé de thiosulfate de sodium puis avec deux fois 30 ml d'eau et séchée sur $MgSO_4$. Après évaporation du solvant, on obtient 44 g (rendement 93%) d'acide bromo-2 (F-octyl)-3 propanoïque.
F : 110°C
Analyse trouvée (calculée) : C 23,12 (23,14) ; H 0,87 (0,71) ; F 56,05 (56,56) ; Br 14,04 (13,99)
IR ( $\nu$ $cm^{-1}$, KBr) ; 1725
$^1H$ RMN ($\delta$ ppm, $CF_3COOH$, TMS) : 4,70 (m, H) ; 3,26 (m, 2H)
SM (LID/IE) m/e (%) : CHBr-$CO_2H$ 137 (52,6) et 139 (55,0) ; M-HF 551 (2,9) and 553 (2,9)

Etape 2 : Préparation de bromo-2 (F-octyl)-3 propanoate d'éthyle (composé n°14)

Un mélange de 22 g (38,45 mM) de l'alpha -bromo-acide composé n°13, de 24 ml d'alcool éthylique et de 6 ml de $H_2SO_4$ concentré est chauffé au reflux pendant 36 heures. Après refroidissement on ajoute 20 ml de $H_2O$. On sépare la phase organique plus basse et on extrait la phase aqueuse deux fois avec 20 ml d'éther diéthylique. On lave les phases organiques combinées avec une solution aqueuse saturée de $K_2CO_3$ puis avec de l'eau puis on sèche sur $MgSO_4$ anhydre. Après élimination du solvant, on isole 20,4 g de l'ester (composé n°14) par distilation ($Eb_{0,4}$=75°C), ce qui correspond à un rendement de 89%.
Microanalyse trouvée (calculée) : C 26,26 (26,04) ; H 1,65 (1,33) ; F 53,97 (53,92) ; Br 13,05 (13,35)
IR ($\nu$ $cm^{-1}$) : 1747
$^1H$ RMN ($\delta$ ppm, $CDCl_3$, TMS) : 4,60 (m, CHBr)

Etape 3 : Préparation de l'azido-2 (F-octyl)-3 propanoate d'éthyle (composé n°15)

Un mélange de 10 g (17 mM) de composé n°14 $C_8F_{17}CH_2CHBrCOOEt$, de 4 g (63 mM) d'azide de sodium et de 20 ml d'éthanol anhydre est chauffé au reflux pendant 24 heures. Après filtration, on concentre la solution alcoolique rouge foncée sous vide. On mélange le résidu avec 25 ml de toluène et on filtre. On sèche le filtrat sur $MgSO_4$ et on le concentre sous vide pour obtenir 7,8 g d'un résidu brun huileux caractérisé par IR comme l'ester azido (rendement 83%). Sa pureté tel qu'indiqué par chromatographie gazeuse est supérieure à 97%.
Microanalyse trouvée (calculée) : C 27,07 (27,82) ; H 1,40 (1,43) ; F 57,40 (57,55) ; N 7,01 (7,48)
IR ($\nu$ $cm^{-1}$) : 1751 ; 2127
$^1H$ RMN ($\delta$ ppm, $CDCl_3$, TMS) : 4,30 (m, $CHN_3$)
$^{19}$ FRMN ($\delta$ ppm, $CDCl_3$, $CFCl_3$) : -113,7 ($\underline{CF_2}$-$CH_2$)

Etape 4 : Préparation de chlorhydrate d'amino-2 (F-octyl)-3 propanoate d'éthyle (composé n°16).

On fait barboter lentement de l'hydrogène dans une suspension agitée de 4,4 g (8 mM) de composé n°15 brut $C_8F_{17}CH_2CH(N_3)COOEt$, et de 200 mg de palladium sur du charbon dans de l'éthanol à la température ambiante. La conversion est complète après 6 heures comme on peut le voir par chromatographie gazeuse. Après filtration, on ajoute 1 ml d'acide chlorhydrique concentré et on isole le chlorhydrate de l'aminoester par filtration, on obtient ainsi 3,3 g de composé n°16, ce qui correspond à un rendement de 74%.
Analyse trouvée (calculée) : C 27,27 (27,29) ; H 2,15 (1,92) ; F 56,12 (56,51) ; N 2,84 (2,44) ; Cl 6,59 (6,21)
IR ($\nu$ $cm^{-1}$) : 1742

Etape 5 : Préparation de chlorure de (N,N,N triméthylammonio)-2 (F-octyl)-3 propanoate d'éthyle (composé n°17)

On dissout 3 g (5,25 mM) du composé n°16 dans 20 ml de MeOH avec 5,42 g de $KHCO_3$. On ajoute goutte à goutte 11,97 g (84,3 mM) d'iodure de méthyle. On laisse le mélange réagir pendant 48 heures à la température ambiante puis pendant 12 heures à 35-40°C. On refroidit ensuite et on filtre le solide en suspension. On évapore le solvant et on lave le solide blanc obtenu avec de l'éther et on le sèche, on obtient ainsi 3,06 g (rendement de 95%) du composé n°17.
IR ($\nu$ $cm^{-1}$, KBr): 2980, 1740, 1465, 1240-1145
$^1H$ RMN ($\delta$ ppm, $CF_3COOH$/TMS) : 1,52 (t, 3H) ; 3,2 (m, 2H) ; 3,52 (s, 9H) ; 4,50 (t, 1H) ; 4,62 (q, 2H).

Etape 6 : Préparation de (N,N,N triméthylammonio)-2 (F-octyl)-3 propanoate (composé n°18).

On dissout 2 g (3,25 mM) du composé n°17 dans 5 ml de MeOH et on ajoute 10 ml d'une solution aqueuse de soude à 10%. On laisse le mélange au reflux pendant 4 heures puis on neutralise avec HCl concentré. On avapore le solvant et on purifie davantage le produit par chromatographie de phase inversée (phase

LiChroprep RP-18, 40 à 63 µm, éluant $MeOH/H_2O$, 80:20) ce qui donne 0,3 g du composé n° 18 (rendement 19%)

Ir (ν $cm^{-1}$, KBr) : 1580, 1400, 1244-1150

Selon und variante on utilise $Me_2SO_4$ comme agent alkylant. Lorsque l'on laisse réagir au reflux pendant une semaine avec 1,5 ml (15,7 mM) de $Me_2SO_4$ et 12 ml d'une solution aqueuse de KOH à 10%, 3 g (5,25 mM) du composé n° 16, on obtient après saponification, neutralisation par HCl et traitement 0,61 g du composé n° 18 (rendement 26%).

EXEMPLES 8 - 9

$C_8F_{17}C_2H_4COCl$ + $HCl.HN(R)-CH_2COOEt$ ⟶ $C_8F_{17}C_2H_4C(O)-N(R)-$ $CH_2CO_2Et$ ⟶ $C_8F_1C_2H_4C(O)-N(R)-CH_2CO_2H$

Composé 19 : R = H ; composé 20 : R = $CH_3$

EXEMPLE 8 : Acide (F-octyl-3′ propylcarbamoyl)éthanoïque (composé n° 19)

$C_8F_{17}C_2H_4COCl$ + $HCl.NH_2-CH_2CO_2Et$ →$C_8F_{17}C_2H_4-C(O)-NH-CH_2CO_2Et$ →$C_8F_{17}C_2H_4-C(O)-NH-CH_2CO_2H$

1,85 g (13 mM) de chlorhydrate du glycinate d'éthyle sont traités par 4 ml (30 mM) de triéthylamine dans 10 ml d'éther éthylique sous argon. 9,12 g (18 mM) de chlorure de (F-octyl)-3 propanoyle sont ajoutés et le mélange réactionnel est agité pendant 4 heures à 25° C, puis filtré. Le filtrat est concentré en une pâte brune. 5,6 g de (F-octyl-3′ propylcarbamoyl) glycinate d'éthyle sont obtenus par distillation (125-130/0,05 mmHg) (Rdt 75%). 2,63 g (4,56 mM) de (F-octyl-3′ propylcarbamoyl) glycinate d'éthyle sont dissous dans 8 ml de NaOH 0,5N et chauffés à reflux pendant une heure. Après acidification avec HCl 1N, l'acide (F-octyl-3′ propylcarbamoyl) éthanoïque (composé n° 19) qui précipite est filtré et recristallisé dans le toluène (2,45 g, Rdt 98%).

F : 110°C

Microanalyse : C trouvé (calculé) : C 28,30 (28,43); H 1,64 (1,47) ; F 58,9 (58,81) ; N 2,40 (2,55)

IR (ν $cm^{-1}$ - KBr) : 1730, 1653, 1558, 1200

RMN¹H (δ ppm, $CD_3COCD_3$, TMS) : 2,61 (m, 4H) ; 3,95 (S, 2H)

RMN¹⁹F (δ ppm, $CD_3COCD_3$, $CFCl_3$) : - 113 ($-CF_2CH_2$)

SM (LID/IC $NH_3$) m/e(%) : M+1 550 (46,1) ; M-COOH 505 (38,7); $C_3F_5^+$ 131 (100)

EXEMPLE 9 : Acide (N-méthyl F-octyl-3′ propylcarbamoyl) éthanoïque (composé n°20)

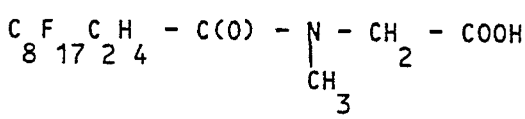

On répète le processus décrit dans l'exemple 8 avec 7,67 g (50 mM) de chlorhydrate de sarcosinate d'éthyle, 33 g (65 mM) de chlorure de (F-octyl-3)propanoyle et 15 ml (150 mM) de triéthylamine. Après distillation, on obtient 21 g (71% de (F-octyl-3′propylcarbamoyl) sarcosinate d'éthyle, et après hydrolyse basique de la fonction ester, 16 g (Rdt 80%) d'acide (N-méthyl-F-octyl-3′ propylcarbamoyl) éthanoïque (composé n°20).

F : 132°C

Microanalyse : C trouvé (calculé) : C 29,38 (29,85) ; H 1,80 (1,79) ; F 57,65 (57,34) ; N 2,48 (2,48)

IR (ν $cm^{-1}$ - KBr) : 1725, 1610, 1210, 1150

RMN¹H (δ ppm, $CD_3OD$, TMS) : 2,70 (m, 4H) ; 3,08 (s, 3H) ; 4,11 (s, 2H)

RMN¹⁹F (δ ppm, $CD_3OD$, TMS) : -113 ($-CF_2CH_2$)

SM (LID/IE) m/e(%) : M-COOH519 (16,6) ; $C_8F_{17}C_2H_4CO^+$ 475 (6) ; $COOH^+$ 44 (100).

EXEMPLE 10 : Acide (F-hexyl-2 éthoxycarbonyl)-4 amino-2 butanoïque (composé n°21)

$C_6F_{13}C_2H_4OH$ + $HOOC-CH_2CH_2CH(NH_2)COOH$ →$C_6F_{13}C_2H_4OC(O)C_2H_4CH(NH_2)COOH$ (composé n°21)

13 g (77 mM) d'acide glutamique, 280 g (770 mM) de F-hexyl-2 éthanol et 20 ml de HCl concentré sont chauffés au reflux pendant 6 heures. Après refroidissement 300 ml d'éther sont ajoutés. Le solide blanc brillant qui précipite est filtré et lavé trois fois à l'éther. L'alcool en excès est récupéré après évaporation du solvant. Le résidu est ensuite versé dans l'eau et le pH est ajusté à 6,5. L'acide (F-hexyl-2 éthoxycarbonyl)-4 amino-2 butanoïque (composé n°21), est isolé par filtration et séché (9,7 g ; Rdt 26%).

F : décomposé à 180°C

Microanalyse : C trouvé (calculé) : C 30,66 (31,66) ; H 2,50 (2,45) ; F 47,98 (50,07) ; N 2,73 (2,84)

IR ( $cm^{-1}$ - KBr) : 1740, 1585, 1520, 1210, 1145

SM (LID/IC $NH_3$) m/e(%) : M 493 (95) ; N-$NH_2$ 476 (100) ; M-COOH 448 (84,7)

EXEMPLES 11 et 12 :
$H_2N(CH_2)_3CH(NH_2)COOH$, HCl ⟶ Cu [$H_2N(CH_2)_3CH(NH_2)COOH$, HCl]$_2$, $2H_2O$ ⟶ Cu [$C_8F_{17}C_2H_4C(O)NH(CH_2)_3CH(NH_2)COOH$]$_2$ (composé n°22)
⟶ $C_8F_{17}C_2H_4C(O)NH(CH_2)_3CH(NH_2)COOH$ (composé n°23)
⟶ $C_8F_{17}C_2H_4C(O)NH(CH_2)_3CH$ [$N^+$ $(CH_3)_3$] $COO^-$ (composé n°24)

EXEMPLE 11 : Acide amino-2 [(F-octyl)-3′ propanoyalamino] -5 pentanoïque (composé n°23)

Etape 1 :
On agite 5 g (30 mM) de chlorhydrade d'ornithine dans 100 ml d'eau avec 13 g (60 mM) de carbonate de cuivre alkalin pendant 1 heure à la température ambiante. On filtre le mélange réactionnel et on lave le solide deux fois avec 50 ml d'eau. On évapore à sec le filtrat bleu et on met le résidu en suspension dans 50 ml d'éthanol. On filtre le solide et on le lave deux fois avec 10 ml d'éthanol. Après séchage, on obtient 6,29 g (rendement 96,5%) de cuivre bis-aquo-bis(ornithine chlorydrate).
F : 210°C
Microanalyse trouvée (calculée) : C 27,07 (27,62) ; H 6,54 (6,44) ; N 11,90 (12,89) ; Cl 16,25 (16,34) ; Cu 14,09 (14,61)
IR (ν $cm^{-1}$-KBr) : 1630, 1605, 1400.

Etape 2 :
On dissout 3 g (7,53 mM) de cuivre bis-aquo-bis(ornithine) monochrlorydrate obtenu dans l'étape précédente avec 4 g (37,7 mM) de $Na_2CO_3$ dans 80 ml d'eau. On ajoute 7,77 g (15,22 mM) de chlorure de l'acide (F-octyl)-3 propanoïque dissous dans 50 ml de toluène. On agite le précité bleu pâle formé pendant 15 heures à la température ambiante, puis on le filtre et on le lave 4 fois avec 50 ml d'eau. Après séchage on lave le solide deux fois avec 50 ml d'éther et on le sèche, ce qui donne 8,89 g (rendement de 93%) de cuivre bis-aquo-bis-acide [(F-octyl)-propanoylamino] pentanoïque (composé n°22).
F : 216°C
Analyse trouvée (calculée) : C 29,51 (29,32) ; H 2,01 (2,44) ; N 3,50 (4,28) ; F 52,26 (49,33) ; Cu 3,70 (4,85)
IR (ν $cm^{-1}$-KBr) : 1645, 1616, 1551

Etape 3 :
On agite 7 g (5,50 mM) du composé n°21 pendant 20 heures à la température ambiante dans un mélange de 110 ml d'une solution aqueuse de EDTA 0,1N et de 55 ml d'acide chlorydrique aqueux 2N. On ajuste le pH du mélange à une valeur de 6-7 par addition de NaOH. On filre le solide et on le lave 5 fois avec 20 ml d'eau. On vérifie l'absence d'ions chlorure dans la dernière fraction d'eau de lavage en ajoutant du nitrate d'argent. On traite de nouveau le solide de la manière ci-dessus avec 90 ml d'une solution de EDTA et 45 ml d'une solution de HCl. Après lavage avec 50 ml d'eau puis avec 20 ml d'acétone, et séchage, on obtient 5,46 g (rendement de 82%) d'acide amino-2 [(F-octyl)-3′ propanoylamino] -5 pentanoïque (composé n°23).
F : 227°C
Analyse trouvée (calculée) : C 30,48 (31,68) ; H 2,09 (2,48) ; N 3,61 (4,62) ; F 54,19 (53,30) ; Cu $<$100 ppm
IR (ν $cm^{-1}$-KBr) : 1647, 1583

EXEMPLE 12 : (triméthylammonio)-2 [(F-octyl)-3′ propanoylamino] -5 pentanoate (composé n°24).
On ajoute 4,63 ml d'une solution aqueuse de KOH à 10% (8,2 mM) à une suspension de 5 g (8,25 mM) d'acide amino-2 [(F-octyl)-3′ propanoylamino] -5 pentanoïque (composé n°23) obtenu comme il est décrit dans l'exemple 11, dans 100 ml de méthanol. On ajoute alors simultanément 3,12 g (24,75 mM, 2,36 ml) de sulfate de diméthyle et 13,9 ml d'une solution aqueuse de KOH à 10% (24, 75mM). Après 5 jours de chauffage au reflux, on ajoute 3,4 ml de HCl concentré. Après chauffage au reflux pendant 24 heures, on filtre la solution, et après addition de 5,21 g (2 mM) de $BaCl_2$ dans 20 ml d'eau, on la centrifuge. On ajuste le pH du surnageant à 7 avec une solution aqueuse de KOH. On évapore les solvants, on sèche le résidu et on sépare le produit par HPLC, ce qui donne 2,49 g (rendement de 46,5%) du composé n°24.
F : 185°C
Analyse trouvée (calculée) : C 31,94 (35,19 ; H 3,26 (3,24) ; N 3,74 (4,32) ; F 43,98 (49,85) : Cl 4,84, 10% en poids (KCl encore présent).
IR (ν $cm^{-1}$-KBr) : 1647, 1558, 1204, 1150
$^{19}F$ RMN (δ ppm, $CD_3OD$, $CCl_3F$) : -114 (-$\underline{CF_2}CH_2$)
SM (LID/IC $NH_3$) m/e (%) : M+1 649 (100).

EXEMPLE 13 : Acide (F-octyl-3′ propylcarbamoyl)-6 amino-2 hexanoïque (composé n°25)
$C_8F_{17}C_2H_4COOH$ + $H_2N(CH_2)_4CH(NH_2)COOH$
→$C_8F_{17}C_2H_4COO^-H_3N^+(CH_2)_4CH(NH_2)COOH$
→$C_8F_{17}C_2H_4C(O)NH(CH_2)_4CH(NH_2)COOH$ (composé n°25)
⟶$C_8F_{17}C_2H_4(CO)NH(CH_2)_4CH[N^+(CH_3)_3]$ $COO^-$ (composé n°26)

25,24 g (51,3 mM) d'acide F-octyl-3 propanoïque sont agités dans 400 ml d'éther avec 5 g (3,2 mM) de lysine pendant 48 heures à température ambiante. Le solide en suspension est filtré et lavé trois fois à l'éther. Après séchage il est placé dans une ampoule scellée sous vide et chauffé à 150°C pendant une nuit. Le solide recueilli est alors lavé trois fois à l'éther et trois fois à l'eau. Après séchage, on obtient 25,4 g (Rdt 80%) d'acide (F-octyl)-3'propyl-carbamoyl)-6 amino-2 hexanoïque (composé n°25).

F : 150°C

Microanalyse : C trouvé (calculé) : C 33,15 (32,90) ; H 2,89 (2,76) ; F 51,19 (50,73) ; N 4,77 (4,51)

IR (ν $cm^{-1}$ - KBr) : 1645, 1582, 1522

SM (LID/IC $NH_3$) m/e(%) : M+1 621 (7,5) ; M-$NH_3$ 603 (77,6) ; M-COOH 575 (6,0) ;

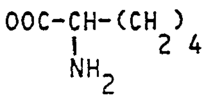 129 (100)

EXEMPLE 14 : (triméthylammonio)-2 [(F-octyl)-3' propanoylamino] -6 hexanoate (composé n°26)

On applique le procédé décrit dans l'exemple 12 à 3 g (4,84 mM) du composé n° 25 obtenu comme il est décrit dans l'exempe 13, dans 50 ml de méthanol, avec 1,83 g (14,52 mM, 1,39 ml) de sulfate de diméthyle. Après HPLC préparative, on obtient 1,26 g (rendement de 39%) du composé n°26.

F : 210°C

Analyse trouvée (calculée) : C 36,34 (36,26) ; H 3,40 (3,50) ; F 46,30 (48,75) ; N 3,96 (4,23)

IR (ν $cm^{-1}$, KBr) : 1645, 1620, 1580, 1202, 1148

$^1$H RMN (δ ppm, $CD_3OD$, TMS) : 1,26-1,55 (1,8H) ; 2,51 (1, 4H) ; 3,20 (s, 9H) 3,62 (t, 1H)

$^{19}$F RMN (δ ppm, $CD_3OD$, $CCl_3F$) : -114(-$CF_2$-$CH_2$-)

SM (LID/IC $NH_2$)m/e (%) : M-(COO, $NMe_3$) 559 (60,0).

EXEMPLE 15 : Acide (F-pentylcarbamoyl)-6 amino-2 hexanoïque (composé n°27)

$C_5F_{11}COOH + H_2N(CH_2)_4CH(NH_2)COOH \rightarrow$

$C_5F_{11}C(O)NH(CH_2)_4CH(NH_2)COOH$ (composé n°27)

13 g (41,4 mM) d'acide F-pentylcarboxylique sont traités par 4 g (27,36 mM) de lysine dans 200 ml d'eau pendant 24 heures. Le mélange réactionnel est lavé à l'éther. Le solide obtenu par lente évaporation de l'eau est placé dans un sublimateur. L'acide (F-pentylcarbamoyl)-6 amino hexanoïque (composé n°27) (Rdt 15%) est recueilli par sublimation (160 C/0,03 mmHg).

F : 150°C

Microanalyse : C trouvé (calculé) : C 31,99 (32,59) ; H 2,96 (2,97) ; F 47,25 (47,34) ; N 6,33 (6,23)

IR (ν $cm^{-1}$ - KBr) : 1680, 1612, 1514, 1202

SM (LID/IC $NH_3$) m/e(%) : M+1 443 (7,2) ; 130 (100) ; 257 (20,0)

EXEMPLE 16 : Acide cholylamino-2 (F-hexyl)-4 butanoïque (composé n°28)

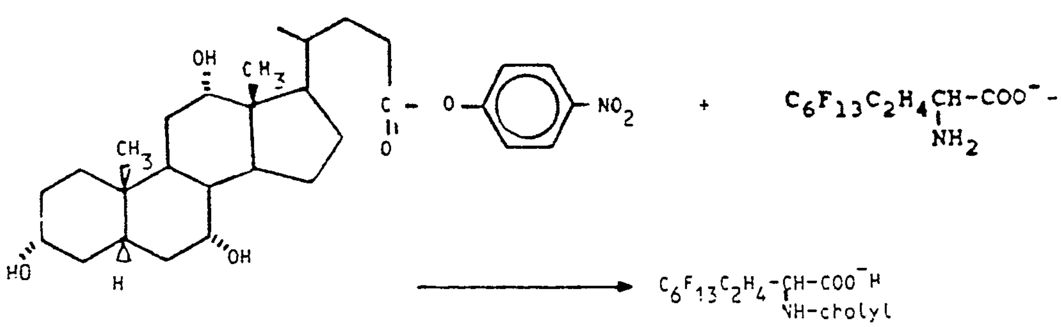

1g (1,9 mM) de cholate de paranitrophényle et 0,85 g (2mM) d'acide amino-2 F-hexyl-4 butanoïque (composé n°6) obtenu dans l'exemple 2, préalablement dissous dans 12ml d'un mélange NaOH 1N/$CH_3OH$ (1:5) sont agités dans du THF et chauffés à reflux pendant 14 heures. Après filtration, la solution est concentrée. Le solide obtenu est dissous dans l'eau et après lavage à l'éther, la phase aqueuse est acidifiée avec HCl 0,1N. Le précipité qui se forme est isolé par centrifugation et séché.

Après chromatographie sur colonne de silice (éluant $CHCl_3$ avec gradient $CH_3OH$), 0,2 g d'acide

cholylamino-2 F-hexyl-4 butanoïque sont obtenus (Rdt 13%) $C_{34}H_{45}F_{13}NO_6$ (composé n°28).
F : 180°C
Analyse trouvé (calculé) : C 46,65 (50,31) ; H 5,31 (5,71) ; F 31,36 (30,43) ; N 1,70 (1,72)
IR (ν $cm^{-1}$- KBr) : 1647, 1597, 1240
RMN$^1$H (δ ppm, $CD_3OD$, TMS) : 0,70 (s, 3H-$C_{18}$) ; 0,90 (s, 3H-$C_{19}$) ; 1,07 (s, 3H-$C_{21}$)
RMN$^{19}$F (δ ppm, $CD_3OD$, $CCl_3F$) : -114 ppm ($CF_2CH_2$)
SM (LID/IE) m/e (%) : 416 (62,2) ; 376 (58,7) ; 253 (100) ; 271 (51,7) ; $CF_3$ (56,1)

EXEMPLE 17 : (F-hexyl)-2 éthylamine (composé n°30)
$C_6F_{13}C_2H_4I$ ---→ $C_6F_{13}C_2H_4N_3$ (cmposé n°29)
---→ $C_6F_{13}C_2H_4NH_2$ (composé n°30)
---→ $C_6F_{13}C_2H_4N(CH_3)_2$ composé n°31)
---→ $C_6F_{13}C_2H_4N(CH_3)_2CH_2COOEt, Cl^-$ (composé n°32)
---→ $C_6F_{13}C_2H_4N(CH_3)_2CH_2COO^-$ (composé n°33)

Etape 1 : Préparation d'azide de (F-hexyl)-2 éthyle (composé n°29).

On chauffe au reflux pendant 15 heures 19 g (40 mM) de $C_6F_{13}C_2H_4I$, 5,4 g (80 mM) de $NaN_3$ et 60 ml de DMF. On verse le mélange réactionnel dans 300 ml d'eau. On sépare la phase inférieure et on lave la phase aqueuse avec de l'éther (2x40 ml). On sèche les phases organiques combinées sur $MgSO_4$ anhydre. Après élimination du solvant on isole par distillation ($Eb_{0,1}$ = 44°C) 13,22 g de l'azide de (F-hexyl)-2 éthyle (composé n°29), ce qui correspond à un rendement de 85%.
Analyse trouvée (calculée) : C 24,45 (24,69) ; H 1,03 (1,03) : F 63,83 (63,47) ; N 10,80 (10,70)
IR (ν $cm^{-1}$) : 2108
$^1$H RMN (δ ppm, $CDCl_3$, TMS) : 3,5 (t, $CH_2N_3$) ; 2,3 (m, $R_FCH_2$)
$^{19}$F RMN : (δ ppm, $CDCL_3$, $CFCl_3$) : -114,4 ($\underline{CF_2}$-$CH_2$)

Etape 2 : Préparation de (F-hexyl)-2 éthylamine (composé n°30)

On fait barboter lentement de l'hydrogène dans une suspension agitée de 8,3 g (21,3 mM) du composé n°29 et 0,81 g de palladium sur du charbon dans 20 ml d'éthanol à la température ambiante. La conversion est totale après 3h30 comme le montre la chromatographie gazeuse. On évapore le solvant pour obtenir 5,7 g (rendement 67%) du composé n°30.
Analyse trouvée (calculée) : C 26,23 (26,47) ; H 1,97 (1,94) ; F 67,12 (67,64) ; N 3,73 (3,85)
IR (ν $cm^{-1}$) : 1607, 3362
SM (LID/IE) m/e (%) : M+H 364 (100) ; M 363 (20) ; $CF_3$ 69 (60)
$^1$H RMN (δ ppm, $CDCl_3$, TMS) : 3,6 (t, $CH_2N$) ; 2,6 (m, $R_FCH_2$)

Etape 3 : Préparation de F-hexyl-2 N,N-diméthyle éthylamine (composé n°31).

On mélange 10 g (27,5 mM) du composé n°30 avec 20 ml (22,7 g, 450 mM) d'acide formique à 88%. On ajoute alors 20 ml d'une solution aqueuse à 37-40% de formaldéhyde et on chauffe le mélange réactionnel pendant 2 heures à 90°C. On ajoute goutte à goutte 10 ml de HCl concentré. On refroidit la solution et on l'extrait avec de l'ester de pétrole. On rend alkaline la phase inorganique par addition d'une solution aqueuse de soude à 50% et on l'extrait avec de l'éther diéthylique. On lave les phases organiques combinées avec 20 ml d'une solution saturée de NaCl, on les sèche sur $MgSO_4$ et on concentre. On distille les 5,6 g d'huile résiduelle orange, ce qui donne 2,36 g de F-hexyl-2 N,N-diméthyl éthylamine (composé n°31) (22% $Eb_{20}$ = 50-50°C).
IR (ν $cm^{-1}$, NaCl) : 2830-2780, 1490, 1240-1145
$^1$H RRMN (δ ppm, $CDCl_3$/TMS) : 2,10 (m, 2H) ; 2,20 (s, 6H) ; 2,6 (t, 2H)

Etape 4 : Préparation de chlorure de N,N-diméthyl, N-(F-hexyl-2 éthyl) ammonio acétate d'éthyle (composé n°32)

1,24 g (3,17 mM) du composé n°31 et 0,5 ml (1,5 équivalent) de chloroacétate d'éthyle sont agités sous reflux dans 2 ml d'éthanol pendant 20 heures. On refroidit le mélange réactionnel et on le verse dans 20 ml d'éther diéthylique. On filtre le précipité, on le lave avec de l'éther diéthylique et on le sèche, ce qui donne 1,23 g (rendement 75%) du composé n°32.
IR (ν $cm^{-1}$, KBr) : 1750, 1240-1145,
$^1$H RMN (δ ppm, $CDCl_3$, $CF_3COOH$/TMS) : 1,46 (t, 3H) ; 2,82 (m, 2H); 3,57 (s, 6H) ; 4,12 (m, 2H) ; 4,41 (s, 2H) ; 4,48 (q, 2H)
Analyse trouvé (calculé) : C 32,62 (32,72) ; H 3,25 (3,31) ; N 2,62 (2,73) ; F 47,08 (48,10) ; Cl 6,67 (6,91)

Etape 5 : N,N-diméthyl, N-(F-hexyl-2 éthyl ) ammonio acétate (composé n°33).

80 mg (2 mM) de NaOH sont ajouté à une solution de 1 g du composé 32 (1,95 mM) dans 10 ml d'éthanol. Le mélange est porté au reflux pendant 4 heures, neutralisé avec HCl concentré, filtré et le filtrat est évaporé à sec. On dissout le résidu dans 1 ml de méthanol. Après addition de 5 ml d'éther diéthylique et refroidissement, on obtient après filtration et séchage 0,25 g du composé n°33 qui est purifié davantage par HPLC (colonne Bondapack $C_{18}$, 30cmx7,8mm, éluant : MeOH/$H_2O$ 70:30) pour donner 0,2 g du composé 33 purifié

(rendement 23%).
IR (ν $cm^{-1}$, KBr) : 1680, 1650, 1390, 1250, 1190, 1150
$^{19}F$ RMN (δ ppm, $CD_3OD/CFCl_3$) : -80,7 (s, 3F) ; -(112,8 (d, 2f) ; -121,2 (s, 2F) ; -122,2 (s, 4F) ; -125,6 (s, 2F)
$^1H$ RMN (δ ppm ; $CD_3OD/TMS$) : 2,83 (m, 2H) ; 3,30 (s, 6H) ; 3,89 (s, 2H) ; 4,05 (m, 2H).
Analyse trouvé (calculé) : C 30,58 (32,54) ; H 2,99 (2,67) ; F 52,06 (55,01 ; N 2,82 (3,12).

EXEMPLE 18 : (F-octyl)-2 éthylamine (composé n°35)

On répète le mode opératoire décrit dans l'exemple 17 avec 10,7 g (18,6 mM) de $C_8F_{17}C_2H_4I$, 4,4 g (67,4 mM) de $NaN_3$ et 50 ml de DMF pendant 20 heures. On isole par distillation ($Eb_{0,1}$:46°C) 5,5 g (rendement 60%) d'azide de (F-octyl)-2 éthyle (composé n°34).
Analyse trouvé (calculé) : C 24,97 (24,55) ; H 0,89 (0,82) ; F 66,10 (66,03) ; N 8,41 (8,59)
IR (ν $cm^{-1}$ : 2109
$^1H$ (δ ppm, $CDCl_3$, TMS) : 3,5 (m, $CHN_3$)
$^{19}F$ RMN (δ ppm, $CDCl_3$, $CFCl_3$) : -114,5 ($\underline{CF_2}$-$CH_2$)

Dans la seconde étape 10,1 g (20,6 mM) du composé n°34 et 800 mg du catalyseur Pd/C conduisent à 5,3 g d'une cire blanche (rendement 60%). Une purification ultérieure par sublimation (65°C, 0,2 mm Hg) donne 4,15 g du composé n°35 pur (rendement 51%).
Analyse trouvé (calculé) : C 25,42 (25,91) ; H 1,32 (1,29) ; F 69,33 (69,76) ; N 4,13 (3,02) pour l'amine libre
IR (ν $cm^{-1}$) : 3300, 1597, 1150
SM (LID/IE) m/e (%) : M-Cl 464 (100) ; M-HCl 463 (72) ; $CF_3$ 69 (55,5) pour le chlorhydrate

Pour illustrer le caractère fortement tensioactif des dérivés fluorés de l'invention, on a effectué des mesures à 20°C de la tension superficielle de solutions dans l'eau de ces dérivés, ainsi que de la tension interfaciale entre ces solutions et un composé perfluoré constitué par la perfluorodécaline.

Les résultats obtenus sont donnés dans le tableau qui suit :

TABLEAU

| Composé | Concentration (g/l) | Tension superficielle ($mN.m^{-1}$) | Tension interfaciale/ F-décaline ($mN.m^{-1}$) |
|---|---|---|---|
| (F-butyl-2')éthyl bétaïne de l'Ex. 4 | 1<br>0,1 | 49,3<br>68,4 | 34,5<br>48,0 |
| (F-hexyl-2')éthyl bétaïne de l'Ex. 5 | 1<br>0,1 | 20,0<br>39,3 | 3,1<br>22,4 |
| (F-octyl-2')éthyl bétaïne de l'Ex. 6 | 1<br>0,1<br>0,01 | 19,1<br>19,9<br>25,6 | 2,8<br>3,4<br>7,5 |
| (F-octyl-3'propyl-carbamoyl)éthanoate de sodium de l'Ex 8 | 1<br>0,1<br>0,01 | 21,6<br>38,8<br>43,2 | 7,1<br>25,2<br>41,3 |
| (N-méthyl F-octyl-3'propyl-carbamoyl)éthanoate de sodium de l'Ex 9 | 0,1 | 34,5 | 18,6 |
| (F-octyl-3''propyl-carbamoyl)-4' butylbétaïne de l'Ex.14 | 0,1<br>0,01 | 19,7<br>22,8 | 3,6<br>5,2 |
| N-cholyl-2 F(hexyl-4-butanoate de sodium de l'Ex.16 | 1 | 28,5 | 4,7 |

On précise que dans les conditions de ces mesures:
- la tension superficielle de l'eau à 20°C est de 73,0± 0,2mN.$m^{-1}$, et
- la tension interfaciale entre l'eau et la perfluorodécaline à 20°C est de 56,0 ± 0,2 mN.$m^{-1}$.

Ainsi, au vu des résultats du tableau ci-dessus, on constate que les dérivés fluorés de l'invention permettent d'obtenir une très forte diminution de la tension à l'interface entre l'eau et l'air ou la perfluorodécaline.

EXEMPLE 19.

Dans cet exemple, on prépare une émulsion comprenant une dérivé fluoré d'acide aminé de l'invention comme agent tensioactif.

L'émulsion est formulée à partir des composés suivants :
- 20% en poids de perfluorodécaline,
- 2% en poids de Pluronic F-68, et
- 1% en poids de (F-octyl-3' propylcarbamoyl) éthanoate de sodium de formule $C_8F_{17}C_2H_4$-C(O)-NH-$CH_2$-COONa

et elle est préparée par sonication.

On compare les propriétés de cette émulsion après un mois à 25°C, à une émulsion de l'art antérieur préparée de façon similaire mais utilisant :
- 20% en poids de perfluorodécaline, et
- 3% en poids de Pluronic F-68.

On constate que dans l'émulsion formulée avec le dérivé fluoré de l'invention, la taille des particules de l'émulsion a augmenté 5 fois moins que dans l'émulsion de l'art antérieur.

Ainsi les nouveaux dérivés fluorés d'acide aminé de l'invention permettent d'améliorer l'émulsification des fluorocarbures.

Pour tester la biocompatibilité des dérivés de l'invention, on a vérifié que ces dérivés n'avaient pas d'effet sur la croissance, la multiplication et le repiquage de cultures cellulaires lymphoblastoïdes de la souche Namalva. En effet, les solutions suivantes :
- à 1 g/l de (F-butyl-2')éthylbétaïne (ex. 4)
- à 1 g/l de (F-hexyl-2')éthylbétaïne (ex. 5),
- à 1 g/l de (F-octyl-2')éthylbétaïne (ex. 6), et
- a 500 mg/l de (F-octyl-3" propylcarbamoyl)-4' butylbétaïne (ex. 14),

dans de l'eau à 9 pour mille de NaCl, ne perturbent pas la croissance, la multiplication et le repiquage de ces cultures.

Des essais effectués sur les globules rouges humains ont montré que des solutions à :
- 5 g/l de (F-butyl-2')éthylbétaïne (ex. 4),
- 5 g/l de (F-hexyl-2')éthylbétaïne (ex. 5),
- 1 g/l de (F-octyl-2')éthylbétaïne (ex. 6), et
- 500 mg/l de (F-octyl-3"propylcarbamoyl)-4'butylbétaïne (ex. 14),

dans de l'eau à 9 pour mille de NaCl ne provoquent pas l'hémolyse des globules rouges humains.

Enfin, on a vérifié que les dérivés de l'invention ne présentaient pas de toxicité aigüe. Ainsi, l'administration intraveineuse à des souris, de 20 ml/kg des solutions à :
- 100 mg/l de (F-butyl-2')éthylbétaïne (ex. 4),
- 100 mg/l de (F-hexyl-2')éthylbétaïne (ex. 5),
- 100 mg/l de (F-octyl-2')éthylbétaïne (ex. 6), et
- 500 mg/l de (F-octyl-3"propylcarbamoyl)-4'butylbétaïne (ex. 14),

dans de l'eau à 9 pour mille de NaCl, a été bien tolérée.

## Revendications

1. Dérivés fluorés d'acides aminés répondant à la formule :

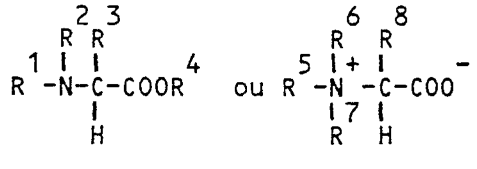

(I) ou (IIa)

dans lesquelles :

- $R^1$ est un atome d'hydrogène, un radical alkyle de 1 à 24 atomes de carbone, un radical dérivé d'un stéroïde ou un radical fluoré de formule $R_F$-$(CH_2)_p$-ou de formule $R_F$-$(CH_2)_p$-C(O)- dans lesquelles p est un nombre entier de 1 à 24 et $R_F$ représente un radical aliphatique perfluoré linéaire ou ramifié, comportant éventuellement dans sa chaîne 1 ou plusieurs atomes d'oxygène, certains atomes de fluor du radical perfluoré pouvant être remplacés par un ou plusieurs substituants choisis parmi les atomes d'hydrogène, de chlore et de brome à condition que le nombre d'atomes de fluor du radical $R_F$ soit supérieur à 7 et que le groupe terminal du radical $R_F$ situé à l'extrémité opposée de celle par laquelle le

radical $R_F$ est lié à $CH_2$ ne comporte pas d'atome d'hydrogène ;
- $R^2$ représente un atome d'hydrogène ou un radical alkyle de 1 à 24 atomes de carbone ;
- $R^3$ représente un atome d'hydrogène ou un radical de formule : $H_2N-C(=NH)-NH-(CH_2)_3-$, ou un radical fluoré de formule :
$R_F-(CH_2)_p-$, $R_F-(CH_2)_n-C(O)-$, $R_F-(CH_2)_p-O-C(O)-(CH_2)_m-$,
$R_F-(CH_2)_n-X^1-(CH_2)_m-$ ou $R_F-(CH_2)_n-X^2-CHR^9-$
dans lesquelles :
- $R_F$ et p ont la signification donnée ci-dessus, m est un nombre entier de 1 à 4, n est égal à 0 ou est un nombre entier de 1 à 24, $X^1$ représente 0 ;S ; -C(O)-NH- ; -C(O)-O- ; $X^2$ représente -O- ou -C(O)-O-, et $R^9$ représente un radical alkyle de 1 à 4 atomes de carbone ou un radical aryle de 6 à 10 atomes de carbone ;
- $R^4$ représente un atome d'hydrogène, un radical alkyle ou un métal physiologiquement acceptable ;
- $R^5$, $R^6$, $R^7$ et $R^8$ qui peuvent être identiques ou différents, représentent un atome d'hydrogène, un radical alkyle ayant de 1 à 24 atomes de carbone, ou un radical fluoré de formule :
$R_F-(CH_2)_p-$, $R_F-(CH_2)_n-C(O)-$, $R_F-(CH_2)_n-X^1-(CH_2)_m-$,
$R_F-(CH_2)_p-O-C(O)-(CH_2)_m-$ ou $R_F-(CH_2)_n-X^2-CHR^9-$
dans lesquelles $R_F$, p, n, m, $X^1$, $X^2$ et $R^9$ ont la signification donnée ci-dessus,
à condition que l'une au moins des radicaux $R^1$ et $R^3$ dans la formule (I) et l'un au moins des radicaux $R^5$, $R^6$, $R^7$ et $R^8$ dans la formule (IIa) soit un radical fluoré.

2. Dérivés fluorés d'acides aminés selon la revendication 1, caractérisés en ce que $R_F$ est choisi parmi les radicaux de formules suivantes :
$F(CF_2)_v-$ avec $4 \leqq v \leqq 12$ ;
$(CF_3)_2CF(CF_2)_w-$ avec $1 \leqq w \leqq 8$ ;
$CF_3-[CF_2CF(CF_3)]_r-$ avec $1 \leqq r \leqq 4$ ;
$C_2F_5-[CF_2CF(CF_3)]_r-$ avec $1 \leqq r \leqq 4$ ;
$(CF_3)_2[CF-CF_2CF(CF_3)]_r-$ avec $1 \leqq r \leqq 4$ ;

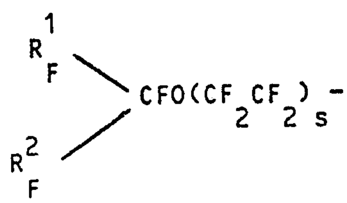

dans laquelle $1 \leqq s \leqq 6$ et $R_F^1$ et $R_F^2$ qui sont identiques ou différents, sont choisis parmi $CF_3-$, $C_2F_5-$, $n-C_3F_7-$, et $CF_3CF_2CF(CF_3)-$
ou dans laquelle $R_F^1$ et $R_F^2$ forment ensemble un radical bivalent choisi parmi $-CF_2(CF_2)_2CF_2-$ et $-CF_2(CF_2)_3CF_2-$ ;
$CF_3CF_2O-(CF_2CF_2O)_t-CF_2-$ avec $O \leqq t \leqq 6$ ; et
$CF_3(CF_2)_2O-[CF(CF_3)F_2O]_u-CF(CF_3)-$ avec $0 \leqq u \leqq 6$.

3. Dérivés fluorés d'acides aminés selon la revendication 1, caractérisés en ce qu'ils répondent à la formule :

$$R^{10}-(CH_2)_p-\underset{\displaystyle NH_2}{\underset{|}{CH}}-COOH \qquad (III)$$

dans laquelle $R^{10}$ est le radical $F(CF_2)_v-$ avec $4 \leqq v \leqq 12$, et $0 < p \leqq 24$.

4. Dérivés fluorés d'acides aminés selon la revendication 1, caractérisés en ce qu'ils répondent à la formule :

$$R^{10}-(CH_2)_p-\underset{\displaystyle \overset{+}{N}(CH_3)_3}{\underset{|}{CH}}-COO^- \qquad (IV)$$

avec $R^{10}$ représentant le radical $F-(CF_2)_v-$ avec $4 \leqq v \leqq 12$, et p tel que $0 < p \leqq 24$.

5. Dérivés fluorés d'acides aminés selon la revendication 1, caractérisés en ce qu'ils répondent à la formule :

$R^{10}-(CH_2)_p-C(O)-NR^{11}-CH_2-COOR^4$ (V)

dans laquelle $R^4$ représente un atome d'hydrogène ou un métal physiologiquement acceptable, $R^{10}$ représente $F-(CF_2)_v-$ avec $4 \leqq v \leqq 12$, $R^{11}$ représente un atome d'hydrogène ou un radical alkyle, de préférence le radical $CH_3$, et $1 \leqq p \leqq 24$.

6. Dérivés fluorés d'acides aminés selon la revendication 1, caractérisés en ce qu'ils répondent à la formule :

$$R^{10}-(CH_2)_p-O-C(O)-(CH_2)_m-\underset{NH_2}{\underset{|}{CH}}-COOH \qquad (VI)$$

dans laquelle $R^{10}$ représente le radical $F-(CF_2)_v-$ avec $4 \leqq v \leqq 12$, p est tel que $1 \leqq p \leqq 24$ et m est égal à 1 ou 2.

7. Dérivés fluorés d'acides aminés selon la revendication 1, caractérisés en ce qu'ils répondent à la formule :

$$R^{10}-(CH_2)_n-C(O)-NH-(CH_2)_m-\underset{NH_2}{\underset{|}{CH}}-COOH \qquad (VII)$$

dans laquelle $R^{10}$ est le radical $F(CF_2)_v$ avec $4 \leqq v \leqq 12$, n est tel que $0 \leqq n \leqq 24$ et m est un nombre entier de 1 à 4.

8. Dérivés fluorés d'acides aminés selon la revendication 1, caractérisés en ce qu'ils répondent à la formule :

$$R^{10}-(CH_2)_n-C(O)-NH-(CH_2)_m-\underset{N(CH_3)_3}{\underset{|+}{CH}}-COO^- \qquad (VIII)$$

dans laquelle $R^{10}$ représente le radical $F-(CF_2)_v-$ avec $4 \leqq v \leqq 12$, $0 \leqq n \leqq 24$ et m est un nombre entier de 1 à 4.

9. Dérivés fluorés d'acides aminés selon la revendication 1, caractérisés en ce qu'ils répondent à la formule:

$$R^{10}-(CH_2)_p-\underset{NHR^{12}}{\underset{|}{CH}}-COO^- \; Na^+ \qquad (IX)$$

dans laquelle $R^{12}$ représente un radical cholyle ou un dérivé du radical cholyle ou apparenté à celui-ci, p est tel que $1 \leqq p \leqq 24$ et $R^{10}$ représente le radical $F(CF_2)_v$ avec $4 \leqq v \leqq 12$.

10. Dérivés fluorés d'acides aminés selon la revendication 1, caractérisés en ce qu'ils répondent à la formule :

$R^{10}(CH_2)_pN^+(CH_3)_2-CH_2)-COOH$ (X)

11. Préparations, solutions, dispersions, gels, émulsions et microémulsions, dans l'eau ou tout autre solvant polaire, de substances et composés non polaires, en particulier hautement fluorés ou perfluorés, caractérisés en ce qu'ils comprennent au moins un dérivé fluoré selon l'une quelconque des

revendications 1 à 10.

12. Préparations selon la revendication 11, caractérisées en ce que les composés hautement fluorés ou perfluorés sont des composés linéaires ou cycliques, ayant des masses moléculaires comprises de préférence entre 400 et 700.

13. Préparations selon la revendication 11, caractérisées en ce que les composés hautement fluorés ou perfluorés sont choisis dans le groupe comprenant :
Les bis(F-alkyl)-1,2-éthènes et plus particulièrement les bis(F-butyl)-1,2-éthènes, les F-isopropyl-1-F-hexyl-2-éthènes et les bis(F-hexyl)-1,2-éthènes, les perfluorodécalines, les perfluorométhyldécalines, les perfluoro-diméthyldécalines, les perfluorométhyl- et diméthyladamantanes, les perfluorodi- et triméthylbicyclo (3,3,1)nonanes et leurs homologues, les éthers de formule :
$(CF_3)_2CFO(CF_2CF_2)_2OCF(CF_3)_2$, $(CF_3)_2CFO(CF_2CF_2)_3OCF(CF_3)_2$,
$(CF_3)_2CFO(CF_2CF_2)_2F$, $(CF_3)_2CFO(CF_2CF_2)_3F$,
$F[CF(CF_3)CF_2O]_2CHFCF_3$, $F[CF(CF_3)CF_2O]_3CHFCF_3$, $(C_6F_{13})_2O$,
les amines $N(C_3F_7)_3$, $N(C_4F_9)_3$, les perfluorométhylquinolidines et perfluorométhylisoquinolidines, les dérivés halogénés $C_6F_{13}Br$, $C_8F_{17}Br$, $C_6F_{13}CBr_2CH_2Br$, bromo-1 perfluoroisopropyl-4 cyclohexane.

14. Préparations selon l'une quelconque des revendications 11 à 13, caractérisées en ce qu'elles comprennent en outre un ou plusieurs autres agents tensioactifs fluorés ou non fluorés.

15. Préparations selon l'une quelconque des revendications 11 à 14, plus particulièrement destinées à servir de transporteurs de gaz, et en particulier de l'oxygène, en milieu vivant, pour des applications en médecine humaine et vétérinaire et en biologie, en particulier comme substituts du sang, agents de contraste pour le diagnostic, milieux pour le traitement de l'ischémie cérébrale et cardiaque, pour la préservation d'organes, de tissus, d'embryons, de semences, milieux utilisables en thérapeutique et chirurgie cardiovasculaire, par exemple comme solution cardioplégique, ou de reperfusion, ou en angioplastie coronaire, milieux utilisables comme adjuvant en radiothérapie et chimiothérapie du cancer, milieux utilisables comme vecteurs de médicaments.

Office européen des brevets

# RAPPORT DE RECHERCHE EUROPEENNE

Numero de la demande

EP 88 40 2310

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | JOURNAL OF ORGANIC CHEMISTRY, vol. 32, février 1967, pages 430-434; N.O. BRACE: "Alpha-amino alkanoic and alkenoic acids with perfluoroalkyl terminal segments"<br>* Pages 430,432-433 *<br>--- | 1,2,3, 11-15 | C 07 C 101/10<br>C 07 C 103/46<br>A 61 K 9/00<br>A 61 K 47/00 |
| X | ANGEWANDTE CHEMIE, vol. 79, no. 17/18, 1967, pages 822-823, Weinheim, DE; W. STEGLICH et al.: "Allgemeine Methode zur Darstellung von beta-Perfluoralkyl-alaninen"<br>* En entier *<br>--- | 1,2,3, 11-15 | |
| X | CHEMICAL ABSTRACTS, vol. 87, no. 9, 1977, page 584, résumé no. 68600x, Columbus, Ohio, US; Y. MAKI et al.: "Synthesis of fluorine-containing DL-alanine derivatives", & YUKI GOSEI KAGAKU KYOKAI SHI 1976, 34(10), 722-5<br>* Résumé *<br>--- | 1-3,11-15 | **DOMAINES TECHNIQUES RECHERCHES (Int. Cl.4)** |
| A | EP-A-0 051 526 (CENTRE NATIONAL DE LA RECHERCHE SCIENTIFIQUE (CNRS))<br>* Revendication *<br>--- | 1-15 | C 07 C<br>A 61 K |
| X | CHEMICAL ABSTRACTS, vol. 93, no. 13, 29 septembre 1980, page 624, résumé no. 132085m, Columbus, Ohio, US; & JP-A-80 35 020 (NEOS CO., LTD) 11-03-1980<br>* En entier * & Tenth Collective Index, vol. 86-95, 1977-1981, Chemical Substances: Gallium-Heptamycin, page 24098CS; N-methyl-N-(3,3,4,4,5,5,6,6,7,7,8,8,8-tridecafluoro-1-oxooctyl<br>--- -/- | 1,2,5, 11-15 | |

Le présent rapport a été établi pour toutes les revendications

| Lieu de la recherche | Date d'achèvement de la recherche | Examinateur |
|---|---|---|
| LA HAYE | 02-01-1989 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 88 40 2310

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DE-A-3 538 836 (MATSUSHITA ELECTRIC INDUSTRIAL) * Page 18, exemple 9 * --- | 1,2,5, 11-15 | |
| X | NATURE, vol. 318, 28 novembre 1985, pages 353-356; E.M. LANDAU et al.: "Transfer of structural information from Langmuir monolayers to three-dimensional growing crystals" * Page 355, tableau 1: monolayer 4 * --- | 1,2,6, 11-15 | |
| X | EP-A-0 102 240 (TEIJIN LTD) * Page 40, lignes 30-33 * --- | 1,2,11-15 | |
| X | FR-A-2 488 700 (FUJI PHOTO FILM CO.) * Page 16: B-26; page 17: B-29 * --- | 1,2,11-15 | |
| X | GB-A-2 062 271 (FUJI PHOTO FILM CO.) * Page 9: (III)-42 * --- | 1,2,11-15 | |
| X | GB-A-2 060 922 (FUJI PHOTO FILM CO.) * Page 9: compound (1)-1 * --- | 1,2,11-15 | DOMAINES TECHNIQUES RECHERCHES (Int. Cl.4) |
| X | US-A-4 126 633 (TOUKAN) * Exemples 1,2 * --- | 1,2,11-15 | |
| X | US-A-3 839 425 (BARTLETT) * Exemples * --- | 1,2,11-15 | |
| X | FR-A-2 207 187 (E.I. DU PONT DE NEMOURS) * Exemples * --- | 1,2,11-15 | |
| X | GB-A- 773 326 (MINNESOTA MINING AND MANUFACTURING CO.) * Page 4, tableau 1; page 6, exemple 4 * --- -/- | 1,2,11-15 | |

Le présent rapport a été établi pour toutes les revendications

| Lieu de la recherche | Date d'achèvement de la recherche | Examinateur |
|---|---|---|
| LA HAYE | 02-01-1989 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 88 40 2310

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DE-A-2 758 013 (CIBA-GEIGY)<br>* Page 34: A25 *<br>--- | 1,2,11-15 | |
| X | CHEMICAL ABSTRACTS, vol. 108, 1988, page 602, résumé no. 140672u, Columbus, Ohio, US; & JP-A-62 189 460 (KONISHIROKU PHOTO INDUSTRY CO., LTD) 19-08-1987<br>*En entier * & Chemical Substance Index, E-I, janvier-juin 1988, page 3525CS; N-(carboxymethyl)-N,N-diethyl-2-[(2,2,3,3,4,4,5,5,6,6,7,7,8,8,9,9,9-heptadecafluoro-1-oxononyl)amino]; N-(carboxymethyl)-2-2[(2,2,3,3,4,4,5,5,6,6,7,7,8,8,9,9,10,10,11,11,11-heneicosafluorol-oxoundecyl)oxy]-N,N-dimethyl<br>--- | 1,2,11-15 | |
| X | CHEMICAL ABSTRACTS, vol. 107, 1987, page 742, résumé no. 165334c, Columbus, Ohio, US; & JP-A-62 06 255 (FUJI PHOTO FILM CO., LTD) 13-01-1987<br>* En entier * & Chemical Substance Index, E-I, juillet-décembre 1987, page 3418CS; N-(carboxymethyl)-N,N-dimethyl-2-2[(2,2,3,3,4,4,5,5,6,6,7,7,8,8,8-pentadecafluorol-oxooctyl)amino]<br>--- -/- | 1,2,11-15 | DOMAINES TECHNIQUES RECHERCHES (Int. Cl.4) |

Le présent rapport a été établi pour toutes les revendications

| Lieu de la recherche | Date d'achèvement de la recherche | Examinateur |
|---|---|---|
| LA HAYE | 02-01-1989 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

RAPPORT DE RECHERCHE EUROPEENNE

Numero de la demande

EP 88 40 2310

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 102, 1985, page 75, résumé no. 133509u, Columbus, Ohio, US; & JP-A-59 187 047 (NIPPON MECTRON CO., LTD) 24-10-1984<br>* En entier * & Chemical Abstracts, Eleventh Collective Index, vol. 96-105, 1982-1986, Chemical Substances: Epoxynaphth-Ethanone; N-(carboxymethyl)-2-[(4,4,5,5,6,6,7,7,8,8,9,9,10,10,11,11,11-heptadecafluoro-1-oxoundecyl)amino]-N,N-dimethyl<br>--- | 1,2,11-15 | |
| X | CHEMICAL ABSTRACTS, vol. 91, 1979, page 346, résumé no. 44852n, Columbus, Ohio, US; H. KUNIEDA et al.: "The lowering of air-water and oil-water interfacial tensions by fluorinated and ordinary hydrecarbon surfactant solutions and spreading of aqueous solutions over the oil surface", & NIPPON KAGAKU KAISHI 1979, (5), 561-7<br>* En entier * & Tenth Collective Index, vol. 86-95, 1977-1981, page 42096CS, Chemical Substances: Pisamin-Propanaminium; N(carboxymethyl)-N,N-dimethyl-3-[(2,2,3,3,4,4,5,5,6,6,7,7,8,8,9,9,10,10,10-nonadecafluoro-1-oxodecyl)amino]<br>--- | 1,2,11-15 | DOMAINES TECHNIQUES RECHERCHES (Int. Cl.4) |
| X | DE-A-2 239 709 (PENNWALT CORP.)<br>* Exemples 4,5 *<br>--- | 1,2,11-15 | |
| X | FR-A-2 014 891 (ALLIED CHEMICAL CORP.)<br>* Exemples 46-48,52; pages 31,33,34; revendications *<br>----- | 1,2,11-15 | |

Le présent rapport a été établi pour toutes les revendications

| Lieu de la recherche | Date d'achevement de la recherche | Examinateur |
|---|---|---|
| LA HAYE | 02-01-1989 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)